# EUROPÄISCHE PATENTSCHRIFT

(11) **EP 2 786 121 B1**
(45) Veröffentlichungstag und Bekanntmachung des Hinweises auf die Patenterteilung: **09.09.2015**
(21) Anmeldenummer: 12810127.6
(22) Anmeldetag: 30.11.2012
(51) Int. Cl.: A61B 5/145, A61B 5/1455, A61B 5/1459, A61B 5/1495, G01N 21/03, G01N 21/27

(54) **MESSKAMMER FÜR EINEN OPTISCHEN SENSOR ZUM BESTIMMEN EINER KONZENTRATION EINES STOFFES IN DER GEWEBEFLÜSSIGKEIT EINES SÄUGERS**
MEASURING CHAMBER FOR AN OPTICAL SENSOR FOR DETERMINING A CONCENTRATION OF A SUBSTANCE IN THE TISSUE FLUID OF A MAMMAL
CHAMBRE DE MESURE POUR UN CAPTEUR OPTIQUE DE DÉTERMINATION DE LA CONCENTRATION D'UNE SUBSTANCE DANS LE LIQUIDE TISSULAIRE D'UN MAMMIFÈRE

(30) Priorität: 02.12.2011 DE 102011087679
(43) Veröffentlichungstag der Anmeldung: 08.10.2014
(73) Patentinhaber: Schildtec GmbH, 99084 Erfurt (DE)
(72) Erfinder: FREITAG, Hans-Joachim, 99084 Erfurt (DE)
(74) Vertreter: Geyer, Fehners & Partner
(86) Internationale Anmeldenummer: PCT/EP2012/074168
(87) Internationale Veröffentlichungsnummer: WO 2013/079704

(56) Entgegenhaltungen:
- WO-A1-2014/026890
- DE-A1-102007 031 284
- US-A1- 2009 277 242

## Beschreibung

Die Erfindung bezieht sich auf eine Meßkammervorrichtung für einen optisch arbeitenden Sensor zum Bestimmen einer Konzentration eines Stoffes, der in Gewebeflüssigkeit eines Säugers enthalten ist, wobei die Meßkammervorrichtung eine Meßkammer aufweist, die zum Einsetzen in den Körper des Säugers ausgebildet ist, mit einem Meßmedium befüllt ist und eine Wandung aufweist, welche zumindest in einem Wandungsabschnitt für den Stoff besser diffusionsdurchlässig ist, als für andere Bestandteile der Gewebeflüssigkeit.

Die Erfindung bezieht sich weiter auf ein Modul für einen Stoff-Konzentrationssensor, das eine Meßkammervorrichtung der genannten Art aufweist.

Die Erfindung bezieht sich weiter auf einen Stoff-Konzentrationssensor, der eine Meßkammervorrichtung der genannten Art aufweist.

Die Erfindung bezieht sich schließlich weiter auf Verfahren zum Herstellen der genannten Meßkammervorrichtung, des genannten Moduls bzw. des genannten Sensors.

Die Messung von Stoffkonzentrationen ist eine häufig zu findende technische Aufgabe. Sie begegnet dann besonderen Schwierigkeiten, wenn der zu messende Stoff in einem Gemisch anderer Stoffe vorliegt. Eine solche Matrix führt dazu, daß für eine gute Konzentrationsmessung nicht nur eine möglichst hohe Empfindlichkeit, sondern auch eine möglichst hohe Selektivität des Meßverfahrens benötigt wird. Oftmals kommt man deshalb mit einer einzigen Messung nicht aus oder muß sehr aufwendige Selektionsmechanismen, wie beispielsweise Gaschromatographie etc. einsetzen. Dabei steigt die Schwierigkeit der Meßaufgabe mit der Komplexität der Matrix.

Sehr komplexe Stoffgemische findet man naturgemäß in der Biologie. Die Messung einer Stoffkonzentration in einer biologischen Matrix ist deshalb eine der aufwendigsten Aufgaben. Grundsätzlich läuft der Meßaufwand den Möglichkeiten zur Miniaturisierung eines Meßsystems zuwider. Miniaturisierte Stoffsensoren sind jedoch besonders für biologische Anwendungen von hohem Interesse. Bekanntermaßen ist es lebensnotwendig, daß beim Menschen einige Stoffe, wie z. B. Glukose, Kochsalz, Harnsäure, Aminosäuren etc., in einer geregelten Konzentration vorliegen. Im Falle einer Erkrankung kann es jedoch zu einer Entgleisung des biologischen Regelkreises kommen, so daß die Stoffkonzentration eines oder auch mehrerer lebensnotwendiger Stoffe außerhalb des physiologisch unbedenklichen Bereiches liegt. Um solch einer Entgleisung durch therapeutische Maßnahmen entgegenzuwirken, muß der Wert der aktuellen Konzentration des (der) entsprechenden Stoffes (Stoffe) dem behandelnden Arzt bekannt sein; die Konzentration muß also gemessen werden, und das mitunter kontinuierlich. Einmal messende Teststreifen sind dafür dann unzureichend.

So nimmt beispielsweise beim Diabetes mellitus, hervorgerufen durch eine gestörte Regulation der Verstoffwechslung der Glukose im Körper, der Glukosespiegel zu hohe (Überzuckerung) oder zu niedrige (Unterzuckerung) Werte an. Dies führt längerfristig zu irreversiblem Absterben von Nervenzellen und ruft eine Reihe krankhafter Veränderungen vor allem an den Blutgefäßen hervor. Folgeerkrankungen, wie Erblindung, Verlust der Nierenfunktion, Herzinfarkt, Bluthochdruck sowie ein Absterben von Gliedmaßen können die Folge sein. Die Diabetes-Therapie erfordert deshalb, daß der Glukosespiegel möglichst präzise und anhaltend auf Werte in einem medizinisch akzeptablen Bereich eingestellt wird, z. B. durch Gabe von Insulin oder Traubenzucker. Zeitpunkt und Menge des zu injizierenden Insulins, oder die Notwendigkeit, Nahrung zu sich zu nehmen, sind dabei von der aktuellen Konzentration der Glukose, wie auch von dem Konzentrationsverlauf während des Tages abhängig.

Die Glukosekonzentration ist somit ein Beispiel für eine Stoffkonzentration in einer komplexen Matrix, die man möglichst kontinuierlich, ohne zeitliche Unterbrechung sowie ohne aufwendig wiederkehrende Anpassungsmaßnahmen kontrollieren möchte. Ausnahmslos alle zur Zeit durchgeführten Therapien widmen sich der Beeinflussung des Blutglukosespiegels, weshalb die meisten Glukosekonzentrationsmeßeinrichtungen auch den Glukosegehalt im Blut bestimmen. Es ist jedoch auch bekannt, die interstitielle Flüssigkeit zu verwenden, da deren Glukosegehalt dem des Blutes mit nur geringer zeitlicher Verzögerung proportional folgt.

So wird beispielsweise in DE 19911265 C2 eine Vorrichtung zur Messung der Glukosekonzentration proteinhaltiger wäßriger Lösungen, insbesondere in interstitiellen Gewebeflüssigkeiten beschrieben, bei der ein Dialysat gleichzeitig polarimetrisch und spektrometrisch analysiert wird. Jedoch ist bei dieser Vorrichtung der technische Aufwand durch den parallelen Einsatz von zwei Meßmethoden enorm groß. Die beschriebene Vorrichtung läßt auch eine große Baugröße erwarten. Des weiteren wird eine aus Kunststoff bestehende Dialysemembran für die Durchführung einer Stofftrennung genannt, jedoch wird deren technisch sehr aufwendige Ankopplung an das optische Meßsystem nicht näher offenbart.

Einen ähnlichen Ansatz offenbart die DE 3736092 A1.

Die US 2009/277242 A1 beschreibt einen faseroptischen Glukosesensor mit einer Sensorspitze, die zum Einstechen in den Körper eines Säugers ausgebildet ist und eine Sensorregion mit einem Glukoserezeptor aufweist. Die Sensorregion wird von einer Membran, die für Glukose diffusionsdurchlässig ist, umhüllt. Ein durch die Bindung von Glukose an den Glukoserezeptor beeinflusstes Fluoreszenzsignal wird durch eine optische Faser zu einem Detektor geleitet. Für eine Mehrpunkt-Kalibrierung des Sensors ist eine Kalibrierkammer mit zwei oder mehr Unterteilungen vorgesehen, die es erlauben, Kalibrierungsmessungen bei zwei oder mehr bekannten Glukosekonzentrationen durchzuführen. Der Sensor und die Kalibrierkammer werden als steriles Kit vorgelegt, indem zunächst die Kalibrierkammer mit Gammastrahlung sterilisiert wird und diese mit dem Sensor in einen Behälter eingebracht wird, der dann mit Ethylenoxid sterilisiert wird.

Möchte man die Stoffkonzentration in einem biologischen Gewebe erfassen, stellt das Gewebe bzw. die Zwischengewebeflüssigkeit eine Matrix dar. Hierzu wird ein Sensor benötigt, der in das Gewebe eingebracht werden kann, da dann eine kontinuierliche Überwachung der Stoffkonzentration möglich ist. Ein solcher Sensor ist aus der DE 102007031284 A1 bekannt. Der dort beschriebene Aufbau realisiert Meßkammer, Sender und Empfänger als kompakte Einheit, die in den Körper eines Säugers eingesetzt werden kann.

Das Einsetzen in den Körper eines Säugers erfordert eine zuverlässige Sterilisierung des Sensors. Beim Aufbau der DE 102007031284 A1 muß auch die im Sensor eingeschlossene Meßflüssigkeit sterilisiert werden. Dies ist nur durch den Einsatz ionisierender (radiaktiver) Strahlung möglich, was allerdings das Problem der Beschädigung des Senders und des Empfängers durch die ionisierende Strahlung aufwirft.

Beim Herstellen eines Sensors gemäß DE 102007031284 A1 muß die Meßkammer blasenfrei befüllt werden, was sich zuverlässig nur mittels Vakuumeinsatz erreichen läßt. Dies erfordert wiederum, daß der komplette Sensor in das Meßmedium eingetaucht werden muß. Bei dieser Art der Vakuumbefüllung muß man natürlich dafür Sorge tragen, daß Sender und Empfänger, insbesondere die dabei verwendete Optik nicht mit dem Meßmedium befüllt werden. Ein hoher Schutzaufwand für diese Elemente ist die Folge.

Die im Sensorkonzept gemäß DE 102007031284 A1 verwendete Meßkammerwandung ist für das Meßmedium oder zumindest einen Teil des Meßmediums diffusionsdurchlässig. Zum Schutz vor Verdunstung bzw. Austritt des Meßmediums ist dort deshalb vorgesehen, den Sensor in einem Lagergehäuse, das vollständig mit dem Meßmedium gefüllt ist, aufzubewahren. Im Falle der Glukosemessung ist das Lagergehäuse mit Kochsalzlösung, die das Meßmedium darstellt, gefüllt. Diese Lagerung bringt hohe und in der Herstellung kostenintensive Anforderungen an den Schutz der Optik- und Elektronikkomponenten des Sensors mit sich. Die elektrischen Leitungen für die Stromversorgung müssen gegenüber der korrosiv wirkenden Kochsalzlösung geschützt werden. Mit der Aufbewahrung in dem Lagergehäuse, welches mit Kochsalzlösung gefüllt ist, ist zugleich auch ein hohes Risiko für die Langzeitstabilität bzw. eine starke Einschränkung der Lagerfähigkeit des Sensors verbunden.

Der Erfindung liegt deshalb die Aufgabe zugrunde, das aus der DE 102007031284 A1 bekannte Konzept dahingehend weiterzubilden, daß die Herstellung und Lagerung vereinfacht ist.

Diese Aufgabe wird erfindungsgemäß gelöst mit einer Meßkammervorrichtung für einen optisch arbeitenden Sensor zum Bestimmen einer Konzentration eines Stoffes, der in Gewebeflüssigkeit eines Säugers enthalten ist, wobei die Meßkammervorrichtung eine Meßkammer aufweist, die zum Einsetzen in den Körper des Säugers ausgebildet ist, mit einem flüssigen Meßmedium befüllt ist und eine Wandung aufweist, welche zumindest in einem Wandungsabschnitt für den Stoff besser diffusionsdurchlässig ist, als für andere Bestandteile der Gewebeflüssigkeit, wobei die Meßkammer aufweist einen ersten Meßkammerfensterabschnitt, der zum Anschluß einer Sendervorrichtung zur Abgabe optischer Strahlung in die Meßkammer vorbereitet ist, und einen zweiten Meßkammerfensterabschnitt, der zum Anschluß einer Empfängervorrichtung zum Empfang von durch die Meßkammer gelaufener optischer Strahlung vorbereitet ist, wobei die Wandung und die Meßkammerfensterabschnitte das Meßmedium tropfdicht einschließen, die Meßkammervorrichtung eine Sterilisierungshülle aufweist, wobei die Sterilisierungshülle zumindest den Wandungsabschnitt der Meßkammer einhüllt, jedoch ohne die Sendervorrichtung und die Empfängervorrichtung einzuhüllen und die Meßkammerfensterabschnitte für die optische Strahlung zu verschließen, die Sterilisierungshülle ebenfalls mit dem Meßmedium gefüllt ist, um den Wandungsabschnitt mit dem Meßmedium zu umgeben, und die Sterilisierungshülle vor dem Einsatz der Meßkammer entfernbar ist.

Die Aufgabe wird weiter gelöst mit einem Modul für einen Stoff-Konzentrationssensor, wobei das Modul gekennzeichnet ist durch eine Meßkammervorrichtung nach einem der obigen Ansprüche, wobei der erste Meßkammerfensterabschnitt dem zweiten Meßkammerfensterabschnitt gegenüberliegt, und die Meßkammer zum Durchlauf der optischen Strahlung ohne Reflexion ausgebildet ist.

Die Aufgabe wird ebenfalls gelöst mit einem Stoff-Konzentrationssensor, gekennzeichnet durch: eine Meßkammervorrichtung nach einem der obigen Ansprüche, einer am ersten Meßkammerfensterabschnitt angeschlossenen Sendervorrichtung und einer am zweiten Meßkammerfensterabschnitt angeschlossenen Empfängervorrichtung.

Die Aufgabe wird auch gelöst durch ein Verfahren zum Herstellen der erfindungsgemäßen Meßkammervorrichtung, wobei a) die Meßkammer mit der Sterilisierungshülle umgeben wird, wobei die Sterilisierungshülle eine Öffnung aufweist, b) die Einheit aus Meßkammer und Sterilisierungshülle unter Einsatz von Vakuum mit dem Meßmedium befüllt werden und die Öffnung verschlossen wird und c) die befüllte Einheit mittel ionisierender Strahlung sterilisiert wird.

Das erfindungsgemäße Konzept sieht also eine eigenständige Meßkammervorrichtung vor, welche erst später, insbesondere erst direkt vor der Anwendung beim Patienten, mit der Sendervorrichtung und der Empfängervorrichtung zum eigentlichen Sensor komplettiert wird. Dieser erfindungsgemäß vorgesehene modulare Aufbau wird dadurch erreicht, daß die Meßkammer die Meßkammerfensterabschnitte aufweist und das Meßmedium einschließt. Zur vereinfachten Sterilisierung und auch zur vereinfachten Lagerung der Meßkammervorrichtung ist die Meßkammer zumindest im Bereich des diffusionsdurchlässigen Wandungsabschnittes mit der Sterilisierungshülle eingehüllt, welche ebenfalls mit dem Meßmedium gefüllt ist. Dadurch kann das Meßmedium aus der Meßkammer während der Lagerung nicht entweichen, beispielsweise verdunsten etc. Zugleich kann die Meßkammervorrichtung problemlos mittels ionisierender Strahlung sterilisiert werden, da die für diese Strahlung empfindlichen Senderund Empfängervorrichtungen auch erst nach der Sterilisierung angebracht werden können. Auch ist das Befüllen der Meßkammer samt Sterilisierungshülle möglich, ohne daß die Empfängervorrichtung und die Sendervorrichtung beim Befüllvorgang angebracht sind, also geschützt werden müßten.

In derart befülltem und durch ionisierende Strahlung sterilisiertem Zustand kann die Meßkammer einfach um die Sendervorrichtung und/oder Empfängervorrichtung ergänzt werden. Das derart erhaltene Modul bzw. der komplette Sensor kann dann unproblematisch oberflächensterilisiert werden, beispielsweise durch eine chemische Sterilisierung, da die Füllung der Meßkammer und der Sterilisierungshülle durch die vorherige Sterilisierung mit ionisierender Strahlung bereits steril ist.

Der modulare Aufbau mit Meßkammervorrichtung, Sendervorrichtung und Empfängervorrichtung erlaubt die Komplettierung in verschiedenen Zuständen. So ist es möglich, daß bereits bei der Produktion die mittels ionisierender Strahlung sterilisierte Meßkammervorrichtung um Sender- und Empfängervorrichtungen komplettiert und diese Einheit dann oberflächensterilisiert wird. Man hat dann einen sterilen Sensor, bei dem vor dem Einsetzen in den Körper lediglich die Sterilisierungshülle entfernt werden muß.

Ein geringerer Grad der Komplettierung ist natürlich möglich. Es hat sich gezeigt, daß bei einem Sensor die in den Körper des Säugers eingesetzten Elemente nach einer gewissen Einsatzdauer ausgetauscht werden müssen. Es sind dies auf jeden Fall die Meßkammervorrichtung und je nach Ausgestaltung auch die Sendervorrichtung. Es ist deshalb unproblematisch möglich, dieses austauchbedürftige Modul separat bereitzustellen. Die mittels ionisierender Strahlung sterilisierte Meßkammervorrichtung wird dann lediglich um die Sendervorrichtung komplettiert und als Modul bereitgestellt. Ein Wechsel dieses Moduls erfolgt dann dadurch, daß das auszutauschende Modul von der Empfängervorrichtung getrennt und durch das neue Modul ersetzt wird. Die Empfängervorrichtung muß dabei nicht ausgetauscht werden.

Wesentlich für das erfindungsgemäß modulare Konzept ist, daß die Meßkammer so ausgebildet ist, daß ihre Wandung und die Meßkammerfensterabschnitte, welche wiederum zum Anschluß von Sender- und Empfängervorrichtung vorbereitet sind, das Meßmedium tropfdicht einschließen. Die Sterilisierungshülle hüllt ihrerseits zumindest die diffusionsdurchlässige Wandung der Meßkammer ein, verschließt jedoch die Meßkammerfensterabschnitte nicht für die optische Strahlung und schließt insbesondere auch nicht die Sendervorrichtung und die Empfängervorrichtung ein. Somit können die Sendervorrichtung und die Empfängervorrichtung an den Meßkammerfensterabschnitten angebracht werden, während die Sterilisierungshülle (noch) die Meßkammer einhüllt.

Die Sterilisierungshülle kann an Bereichen der Meßkammer dichtend angebracht werden, welche die Meßkammerfensterabschnitte umgeben. Die Sterilisierungshülle überdeckt die Meßkammerfensterabschnitte dann nicht. Die Bereiche, an denen die Sterilisierungshülle angebracht ist, können beispielsweise entsprechende Vertiefungen, Aussparungen oder Nuten an den Meßkammerfensterabschnitten sein. Ein mögliches Beispiel ist ein stufenförmiger Kragen an den entsprechenden Meßkammerfensterabschnitten, an denen die Sterilisierungshülle mit ihrem Rand angebracht wird.

Alternativ zum Freilassen der Meßkammerfensterabschnitte kann die Sterilisierungshülle auch für die optische Strahlung transparent ausgestaltet sein und die Meßkammerfensterabschnitte überdecken. In diesem Überdeckungsbereich verbleibt die Sterilisierungshülle dann am Sensor, auch nachdem die Sender- und Empfängervorrichtungen an den Meßkammerfensterabschnitten angebracht wurden. Die Transparenz muß nicht durchgehend gegeben sein. Es genügen u. U. Fensterbereiche in der Hüllenwand.

Die Hüllenwand der Sterilisierungshülle kann besonders einfach unter Verwendung einer Folie realisiert werden, die zum Entfernen der Sterilisierungshülle abgerissen wird. Hierzu können Aufreißfäden vorgesehen werden.

Die Meßkammerfensterabschnitte und die Wandung schließen das Medium tropfdicht in der Kammer ein. Eine geeignete Verbindung zwischen Meßkammerfensterabschnitten und Wandung ist deshalb erforderlich. Hierfür bieten sich Verklebungen an. Eine Verklebung hat jedoch den Effekt, daß sie die Diffusionspermeabilität der Wandung zumindest im Verklebungsbereich herabsetzt, da Klebstoff in die Wandung eindringt. Bereiche der Wandung, in denen die Diffusionspermeabilität durch die Verklebung herabgesetzt sind, finden sich üblicherweise nahe der Kontaktstelle zwischen Meßkammerfensterabschnitten und Wandung. Um eine Verfälschung der Messung durch solche Bereiche zu vermeiden, ist es bevorzugt, daß die Meßkammerfensterabschnitte in die Meßkammer hineinragen und diese Bereiche überdecken. Sie stehen somit zur Diffusion nicht zur Verfügung. Die Änderung der Diffusionseigenschaften in diesem Bereich führt deshalb nicht zu einer Verfälschung der Messung.

Die Befestigung von Sender- und/oder Empfängervorrichtung an den Meßkammerfensterabschnitten kann auf verschiedenste Art erfolgen, beispielsweise durch eine Verklebung. Im Hinblick auf einen modularen Einsatz der Meßkammervorrichtung ist jedoch ein Befestigungsmittel zum Befestigen des anzuschließenden Senders bzw. Empfängers zu bevorzugen, das gleichzeitig eine präzise Lagejustierung bewirkt. Dies kann beispielsweise durch eine Verklebung oder Verkittung in Kombination mit geeigneten Justierstrukturen erreicht werden. Auch möglich ist ein Flansch für die Befestigungsmittel, an welchen die Sender- und Empfängervorrichtung angebracht werden.

Das Anbringen von Sender und Empfänger an den Meßkammerfensterabschnitten muß selbstverständlich so erfolgen, daß die optische Strahlung in die Meßkammerfensterabschnitte ein- bzw. daraus ausgekoppelt werden kann. Neben einer die optischen Anforderungen erfüllenden Verklebung oder Verkittung ist es auch möglich, ein Reservoir für eine Immersionsflüssigkeit vorzusehen, um den Durchtritt der optischen Strahlung durch die Verbindungsstelle sicherzustellen. Dies kann beim Anschluß der Sendervorrichtung und/oder beim Anschluß der Empfängervorrichtung verwendet werden.

Sender und/oder Empfänger können auch über eine Lichtleitfaser angeschlossen werden. Hierfür wird vorzugsweise eine polarisationserhaltende Single-Mode-Fiber verwendet, wenn eine Polarisationsauswertung erfolgen soll. Zum Anschluß von Sender und/oder Empfänger über die Lichtleitfaser ist es bevorzugt, den entsprechenden bzw. die entsprechenden Meßkammerfensterabschnitt(e) als Faserkoppler auszubilden. Die Ankopplung von Sender und/oder Empfänger über die Lichtleitfaser hat den Vorteil, daß Sterilisierungsanforderungen leichter erfüllbar sind. Zudem ist die Lage von Sender und Empfänger dadurch variabler. Der Begriff "Fensterabschnitt" umfaßt also auch einen Faserkoppler.

Wie bereits aus dem Stand der Technik bekannt ist, sollte ein Sensor vor dem Einsatz kalibriert werden, wobei ein Zweipunkt-Kalibrierung zu bevorzugen ist. Im Hinblick auf die Lagerungsfähigkeit des Sensors ist es günstig, wenn diese Kalibrierung möglichst direkt vor dem Einsatz des Sensors vorgenommen werden kann. Eine Zweipunkt-Kalibrierung erfordert, daß die Meßkammer nacheinander mit zwei Meßmedien befüllt wird, welche sich in ihrer Zusammensetzung, insbesondere im Gehalt eines zu messenden Stoffes, unterscheiden. Der Stoff kann beispielsweise Glukose sein. Es ist deshalb bevorzugt, die Sterilisierungshülle der Meßkammer mit einer Kalibrierkammer über eine Fluidverbindung zu verbinden, wobei die Kalibrierkammer ein Kalibriermittel enthält. Die Fluidverbindung kann geöffnet werden, um für Kalibrierzwecke das Kalibriermittel mit dem Meßmittel zu mischen. Damit können die zwei Meßpunkte, welche für die Kalibrierung verwendet werden, einfach realisiert werden. Der erste Meßpunkt wird durch Verwendung des bei der Herstellung in die Meßkammer eingefüllten Meßmittels verwirklicht. Der zweite Kalibrierpunkt wird dadurch erreicht, daß die Sterilisierungshülle mit der Kalibrierkammer fluidisch verbunden ist/wird, insbesondere eine schon vorhandene Fluidverbindung geöffnet wird. Dadurch mischt sich das Kalibriermittel mit dem Meßmittel. Dieses Gemisch dient zur Erzeugung eines zweiten Meßpunktes. Das Verhältnis der Füllvolumina von Meßkammer und Kalibrierhülle und die entsprechenden Konzentrationen sind natürlich für die Kalibrierung relevant.

Die Befestigung eines Sensors, der in dem Körper eines Säugers eingebracht wird, ist natürlich dann von besonderer Relevanz, wenn Teile des Sensors sich weiterhin außerhalb des Körpers befinden. Dies ist beim hier betrachteten Sensorkonzept der Fall, bei dem sich regelmäßig die Meßkammer innerhalb des Körpers des Säugers befindet, die Empfängereinrichtung und die zumindest die auswertende Elektronik jedoch außerhalb. Eine besonders schonende Lagefixierung erreicht man, indem an der Meßkammer flexible Befestigungsmittel, beispielsweise in Form einer Manschette vorgesehen sind, welche an der Haut des Säugers befestigbar sind. Die in den Körper ragenden Teile des Sensors sind dann flexibel gelagert, so daß sie Körperbewegungen etc. folgen können.

Über dem Teil des Sensors, der aus dem Körper ragt, üblicherweise die Empfängervorrichtung, wird zweckmäßigerweise eine Schutzkappe angebracht, die unabhängig von den Befestigungsmitteln für die Meßkammer an der Haut befestigt wird. Eine Berührung, Druck oder sonstige Krafteinwirkung auf die Schutzhülle führt so nicht zu einer schmerzhaften Bewegung des in den Körper ragenden Teils, da keine starre Verbindung zwischen der Schutzkappe und den Teilen des Sensors besteht, die in den Körper einbracht sind bzw. mit eingebrachten Teilen verbunden sind.

Für die Funktion der Meßkammervorrichtung ist eine möglichst blasenfreie Befüllung des Meßkammerinneren, wie bereits erwähnt, möglichst anzustreben. Um eventuelle Restblasen in der Meßkammer tolerieren zu können, ist es vorteilhaft, bei mindestens einem Meßkammerfensterabschnitt die zum Meßkammerinneren zeigende Fläche mindestens konvex auszubilden. Durch diese konvexe Ausbildung werden Gasbläschen nach außen gedrückt und stören so die optische Durchstrahlung der Meßkammer nicht oder zumindest weniger. Prinzipiell genügt es, denjenigen Meßkammerfensterabschnitt konvex auszugestalten, der beim späteren Einsatz des Sensors oben liegt, zu dem also eventuelle Gasbläschen nach oben steigen. Ist die spätere Orientierung der Meßkammer nicht festgelegt, ist es zu bevorzugen, beide Meßkammerfensterabschnitte konvex auszubilden. Diese konvexe Ausbildung kann unabhängig von der Sterilisierungshülle vorteilhaft eingesetzt werden.

Durch den Brechzahlunterschied zwischen Material des Meßkammerfensterabschnittes und dem Medium, können die konvexen Flächen optional zusätzlich als optische Flächen zur Strahlformung ausgebildet und genutzt werden. Damit erreicht man eine bessere Fokussierung des Lichtstrahles bzw. kann störende Randstrahlen vermeiden.

Die Meßkammervorrichtung kann, wie bereits erwähnt, zu einem Modul für einen Stoff-Konzentrationssensor ergänzt werden, das zum Einsetzen in den Körper des Säugers vorbereitet ist, indem die Sendervorrichtung am ersten Meßkammerfensterabschnitt angeschlossen wird. Wenn die Meßkammer zum einmaligen Durchlauf der optischen Strahlung ausgebildet ist, also erster und zweiter Meßkammerfensterabschnitt sich an gegenüberliegenden Enden der Meßkammer befinden, umfaßt dieses Modul alle Bestandteile, welche in den Körper eingebracht werden. Dies ist die üblicherweise zu bevorzugende Anordnung, da sie zu einem sehr schlanken Modul führen. Dennoch ist es als Option möglich, in der Meßkammer eine Reflexionsstelle für die optische Strahlung vorzusehen, so daß sich erster und zweiter Meßkammerfensterabschnitt nicht an gegenüberliegenden Enden der Meßkammer befinden, sondern beispielsweise nebeneinander liegen. Dies hat den Vorteil, daß Sender- und Empfängervorrichtung gemeinsam außerhalb des Körpers verbleiben können, insbesondere zu einer gemeinsamen Einheit zusammengefaßt werden können.

Um Störstrahlung möglichst zu eliminieren, ist es zu bevorzugen, die Schutzkappe und/oder die flexiblen Befestigungsmittel aus optisch nicht transparentem Material zu fertigen.

Gemäß dem erfindungsgemäßen Konzept wird zumindest die Meßkammer, vorzugsweise das beschriebene Modul, in den Körper des Säugers eingestochen. Dabei sollte die Meßkammer möglichst vollständig unter der Haut liegen. Dies läßt sich natürlich einfach mit einem nahezu senkrechten Einstechen erreichen. Um jedoch das Verletzen von Organen oder Blutgefäßen etc. zu vermeiden, ist ein schräges Einstechen vorteilhaft. Um auch dann sicherzustellen, daß die Meßkammer sich möglichst vollständig unter der Haut, d. h. im Gewebe des Säugers befindet, ist es zu bevorzugen, den bzw. diejenigen Meßkammerfensterabschnitt(e) in Form einer Verlängerung auszugestalten, an welchem die Sender- bzw. Empfängervorrichtung angeschlossen wird, die außerhalb des Körpers verbleibt. Dies erlaubt es auch bei flachem Einstichwinkel die Meßkammer vollständig in das Gewebe einzubringen.

Durch die Diffusionsabtrennung des Stoffes aus der Matrix (Gewebeflüssigkeit) löst das Sensorprinzip das im Stand der Technik bestehende Problem der geringen Spezifizität, mit dem eine rein physikalische Meßmethode meist behaftet ist. Durch die diffusionsbedingte Abtrennung des Stoffes aus der Matrix kommt der Sensor mit einem einfachen optischen Meßaufbau aus und ist somit sehr kompakt. Die Wandung selektiert durch ihre Diffusionseigenschaften den Stoff aus der Matrix und reichert ihn in der Meßkammer an, indem er von anderen Matrixbestandteilen getrennt wird. Die Diffusionstrennung kann dabei durch eine Größen- und/oder eine Formselektion erfolgen, d.h. die Wandung läßt nur Stoffe in einen bestimmten Größenbereich oder einem bestimmten Formbereich ihrer Moleküle passieren.

Durch die diffusionsabtrennende Funktionalität des Sensors wird das optische Meßverfahren wesentlich vereinfacht, was eine kompakte, miniaturisierbare und kostengünstige Umsetzung ermöglicht. Insbesondere kann durch die Diffusionsabtrennung verhindert werden, daß Substanzen, die auf das verwendete optische Meßverfahren ebenfalls und möglicherweise in einem viel stärkeren Maß als der nachzuweisende Stoff ansprechen, in die Meßkammer gelangen.

Der von der Sendervorrichtung erzeugte elektromagnetische, optische Meßstrahl (z. B. mit Wellenlängen zwischen 0,8 und 1,5 µm) durchstrahlt die Meßflüssigkeit. Über Polarisationszustände (un-, teil-, linear-, elliptisch- oder zirkularpolarisiert) und/oder Wellenlängenverteilung des Meßstrahls ist es möglich, die Art des Meßverfahrens (Polarimetrie oder Absorptions- oder Streulichtmessung) vorzugeben und somit den Sensor auf einen oder mehrere Stoffe auszulegen. Der Meßstrahl trifft nach Durchlauf durch das Meßmedium auf die Empfängervorrichtung, die vorzugsweise aus mindestens zwei voneinander unabhängigen Detektoren besteht, denen ein Strahlteiler vorgeschaltet ist. Dadurch ist eine umfangreiche Meßstrahlanalyse möglich. Liegt der Empfänger dem Sender gegenüber, wird nur ein Durchgang durch das Meßmedium benötigt, was zu einem schlanken Aufbau führt.

Die Diffusionseigenschaften der Wandung sind vorzugsweise so gewählt, daß eine gute Diffusion und damit ein guter Durchtritt nur für den zu messenden Stoff, nicht jedoch für andere Substanzen der zu messenden Matrix gegeben ist. Es ist also durchaus im Rahmen der Erfindung auch möglich, bei der Auslegung des Sensors die Diffusionseigenschaften der Wandung passend zur Matrix und dem Stoff vorzugeben bzw. einzustellen.

Die Diffusionseigenschaften der Wandung sorgen für die gewünschte Selektivität des Sensors, so daß die in der Meßkammer stattfindende optische Messung eine hohe spezifische Genauigkeit und eine niedrige Nachweisgrenze für den Stoff erreicht, ohne daß aufwendige optische Aufbauten nötig sind. Auch entfällt durch die Diffusionsselektion das Bedürfnis für eine lange Meßstrecke, wodurch der Aufbau kompakt gehalten werden kann.

Die Meßkammervorrichtung kann vorzugsweise ein längliches Gehäuse, z. B. ein Kapillarröhrchen, aufweisen, an dessen Stirnseiten die Meßkammerfensterabschnitte angebracht sind. Der Querschnitt des länglichen Gehäuses trägt zur Meßgenauigkeit nicht bei, wodurch ein schmales Gehäuse realisierbar ist, beispielsweise mit einem Durchmesser unter 3 mm, das als Einstichsonde arbeitet.

Die optische Messung in der Meßkammer wird natürlich passend zum nachzuweisenden Stoff sowie zum Meßmedium in der Meßkammer gewählt. Eine mögliche optische Messung ist ein photometrisches Verfahren. Photometrische Methoden zeichnen sich gegenüber anderen analytischen Verfahren durch hohe Empfindlichkeit, Einfachheit und die Möglichkeit von großen Reihenversuchen unter standardisierten Bedingungen aus. Für eine quantitative Analyse durch Absorptionsphotometrie benutzt man z. B. ultraviolette oder sichtbare Strahlung. Dieser Spektralbereich entspricht Änderungen in der Energie der Valenzelektronen. Möglich ist auch die Nutzung des infraroten Spektralbereiches, bei dem in Molekülen des nachzuweisenden Stoffes Veränderungen der Kernschwingungsenergie erfolgen.

Allerdings zeigt nur ein geringer Teil der zu untersuchenden Substanzen Absorptionsbanden im Licht (Farbe) oder im ultravioletten Bereich. In den meisten Fällen ist es aber möglich, den Stoff durch geeignete chemische Reaktionen in eine charakteristisch absorbierende Verbindung zu überführen und somit deren Konzentration zu bestimmen. Seit Einführung photometrischer Meßverfahren sind weit über 1000 Analysemethoden beschrieben worden, die prinzipiell alle hier eingesetzt werden können. Die chemische Reaktion kann dadurch eingeleitet werden, daß der Stoff in die Flüssigkeit der Kammer eindiffundiert. Beispielsweise kann nach entsprechender Aufarbeitung folgendes nachgewiesen werden: Acetonkörper, Bilirubin, Cholesterin, Eisen, Gallensäure, Hämoglobin, Harnsäure, Kohlenmonoxid, Reststickstoff im Blut, etc.

Das flüssige Meßmedium in der Meßkammer ist somit mitunter abhängig von Stoff und/oder von der Matrix zu wählen. Insbesondere kann sie so gewählt sein, daß es den zu messenden Stoff in einer Normkonzentration enthält. Das Empfängersignal zeigt dann Abweichungen von der Normkonzentration an.

Als erfaßte Meßgröße kommt die Abschwächung der Strahlung beim Durchgang durch das Meßmedium in Frage, wobei auch eine wellenlängen- oder polarisationsselektive Auswertung erfolgen kann. Auch können mehrere Strahlen bei verschiedenen Wellenlängen ausgewertet werden. Es ist dazu bevorzugt, daß die Sendervorrichtung eine Strahlungsquelle und ein optisches Filtersystem oder ein Abbildungssystem oder beides aufweist. Das Abbildungssystem sorgt für möglichst optimalen Durchtritt der Strahlung durch die Meßkammer und paßt insbesondere die optische Strahlung, die von der Strahlungsquelle emittiert wird, an den Querschnitt und die Länge der Meßkammer an. Es kann dazu beispielsweise eine Kollimatoroptik umfassen. Die Strahlungsquelle kann als Leuchtdiode, Laserdiode oder Leuchtdiodenarray ausgebildet sein.

Das Filtersystem ist auf den auszuwertenden optischen Effekt abgestimmt, der beispielsweise Breitbandabsorption, wellenlängenselektive Absorption, polarisationsabhängige Absorption oder Polarisationsdrehung umfassen kann. Es ist deshalb zweckmäßig, daß das Filtersystem ein Polarisationsfilter und/oder ein Interferenzfilter und/oder ein Kantenfilter umfaßt.

Eine besonders hohe Meßgenauigkeit erreicht man, wenn empfängerseitig eine Differenzanalyse erfolgt oder zwei unterschiedliche optische Effekte ausgewertet werden. Es ist deshalb bevorzugt, daß die Empfängervorrichtung mindestens zwei fotoempfindliche Detektoren und mindestens ein optisches Filtersystem aufweist, das zum senderseitigen Filtersystem paßt.

Das Meßmedium wird, wie bereits erwähnt, vorzugsweise passend zur Matrix gewählt. Bei einem biologischen Gewebe bietet sich als Meßmedium eine physiologische Kochsalzlösung oder eine Glukoselösung an. Bei Auswertung der Polarisationsdrehung ist es zu bevorzugen, daß das senderseitige Filtersystem einen Polarisationsfilter aufweist, das empfängerseitige Filtersystem ebenfalls einen Polarisationsfilter aufweist und die Auswerteschaltung die Polarisationsdrehung der Strahlung beim Durchgang durch die mit dem Meßmedium gefüllte Meßkammer ermittelt und daraus die Konzentration des Stoffes ableitet.

Es versteht sich, daß die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine Schemadarstellung einer Meßkammervorrichtung für einen Sensor zur Messung einer Stoffkonzentration in einer Flüssigkeit,
- Fig. 2-4: Abwandlungen der Bauweise der Fig. 1,
- Fig. 5: einen Sensor mit einer Meßkammervorrichtung gemäß Fig. 1 in einem lagerfähigen Zustand und noch nicht vorbereitet für das Einstechen am Patienten,
- Fig. 6: eine Darstellung ähnlich der Fig. 5, hier nun betreffend einer Weiterbildung, die zu einer Zweipunkt-Kalibrierung ausgebildet ist,
- Fig. 7: ein Modul für einen Sensor ähnlich der Fig. 1, jedoch ohne Empfangsvorrichtung,
- Fig. 8: eine abgewandelte Bauweise des Moduls der Fig. 7 und
- Fig. 9: eine Schemadarstellung eines Sensors verwendend das Modul der Fig. 8, der unter die Haut eines Patienten gestochen und um weiter Bauteile vervollständigt ist.

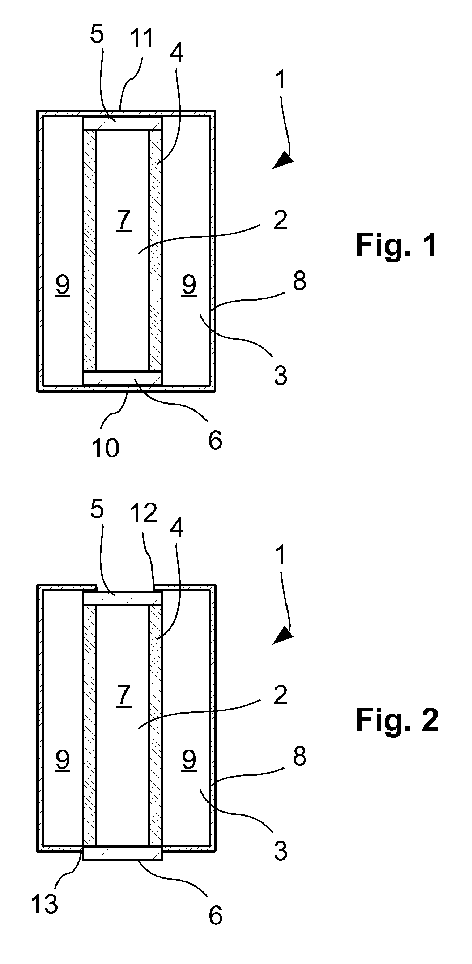

Fig. 1 zeigt schematisch einen Längsschnitt durch eine Meßkammervorrichtung 1 für einen Sensor, der das Meßprinzip gemäß DE 102007031284 A1 realisiert. Abweichend zum dort beschriebenen Sensor ist hier jedoch eine modulare Bauweise eines Sensors vorgesehen, die aus der Meßkammervorrichtung 1 sowie noch zu beschreibenden Sender- und Empfängervorrichtungen besteht. Hinsichtlich des Meßprinzips wird voll umfänglich auf die DE 102007031284 A1 verwiesen.

Die Meßkammervorrichtung 1 weist eine Meßkammer 2 auf, welche mit einem flüssigen Meßmedium (z. B. physiologische Kochsalzlösung) befüllt ist. Die Meßkammer ist von einer Sterilisierungshülle 3 umgeben, welche ebenfalls mit dem Meßmedium befüllt ist. Die Meßkammer 2 ist durch ein längliches Röhrchen, z. B. durch eine Kapillare 4, gebildet, das an seinen Stirnseiten mit Fenstern 5 bzw. 6 verschlossen ist. Dadurch ist ein Meßkammerinnenraum 7 gebildet, in dem das Meßmedium tropfdicht eingeschlossen ist. Unter dem Begriff "tropfdicht" wird dabei verstanden, daß das Meßmedium nicht aus dem Meßkammerinnenraum 7 hinausfließen kann, eine Diffusion durch die Wandung des Röhrchens (z. B. der Kapillare 4) jedoch möglich ist.

Die Sterilisierungshülle 3 hüllt die Wandung mit einer Hüllenwand 8 ein, so daß zwischen Kapillare 4 und Hüllenwand 8 einen Hülleninnenraum 9 gebildet ist. Dieser ist ebenfalls mit dem flüssigen Meßmedium befüllt.

Die Hüllenwand 8 ist so ausgebildet, daß sie die Fenster 6 und 5 durch Hüllenfenster 10 und 11 überdeckt. Die Fenster 5 und 6 sowie die entsprechenden Hüllenfenster 11 und 10 sind für die optische Strahlung transparent, die durch den Meßkammerinnenraum 7 laufen soll.

Die Hüllenwand 8 und die Sterilisierungshülle 3 bewirken, daß die Wandung der Kapillare 4 beiderseitig von dem flüssigen Meßmedium umgeben ist. Die Befüllung des Meßkammerinnenraums 7 sowie des Hülleninnenraums 9 erfolgt vorzugsweise durch eine Vakuumbefüllung. Dazu wird eine kleine Öffnung in der Hüllenwand 8 vorgesehen, die Meßkammervorrichtung 1 evakuiert und anschließend mit dem flüssigen Meßmedium, beispielsweise Kochsalzlösung befüllt. Nach dem Befüllvorgang wird die Befüllungsöffnung in der Hüllenwand 8 geschlossen. Alternativ kann in der Hüllenwand 8 auch ein Befüllventil vorgesehen werden.

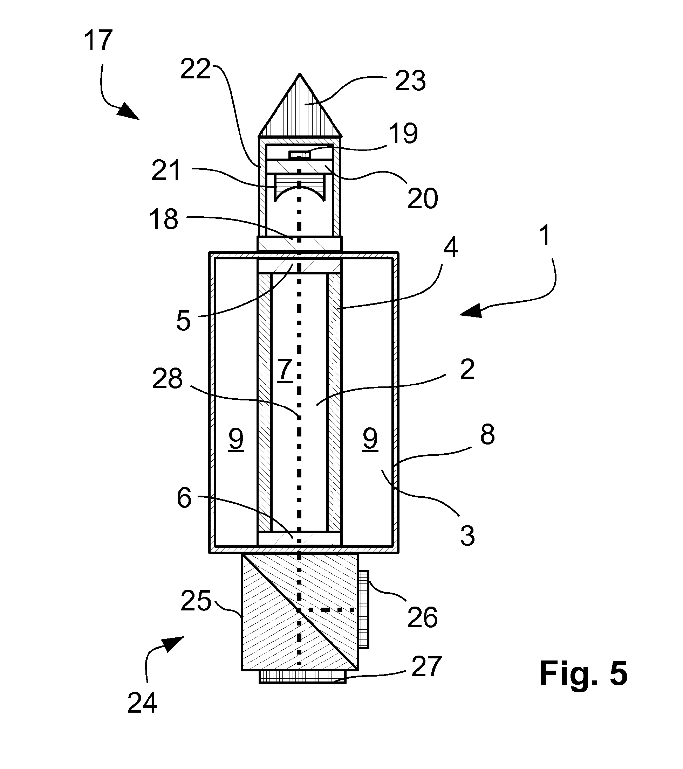

Die Meßkammervorrichtung 1 ist dafür ausgebildet, daß mit den Fenstern 5 und 6 eine Sendervorrichtung bzw. eine Empfängervorrichtung des Sensors verbunden wird. Den entsprechenden Zustand zeigt Fig. 5, anhand der der Aufbau noch erläutert werden wird. Wesentlich für die Bauweise der Fig. 1 ist, daß die Hüllenwand 8 die Fenster 5 und 6 nicht abdeckt. Sie weist dazu in der Bauweise der Fig. 1 die entsprechenden Hüllenfenster 11 und 10 auf. Die optische Sendevorrichtung sowie die optische Empfangseinrichtung kann somit direkt auf den Hüllenfenstern 11 und 10 angebracht werden.

Fig. 2 zeigt diesbezüglich eine Abwandlung. Hier ist das Fenster 5 von der Hüllenwand 8 ausgespart. Diese weist einen Hüllenrand 12 auf, der auf dem Rand des Fensters 5 befestigt ist. Eine weitere optionale Ausgestaltung findet sich in Fig. 2 exemplarisch am Fenster 6. Hier ist der Hüllenrand 13 am seitlichen Rand des Fensters 6 befestigt.

In einer nicht weiter dargestellten Option sind die Fenster 5 und 6 als Faserkoppler zum Anschließen einer Lichtleitfaser ausgebildet, über welche die Sendereinrichtung bzw. die Empfangseinrichtung angekoppelt wird. Die Hüllenwand 8 ist dann mit diesem Faserkoppler verbunden. Der Faserkoppler ist somit an den außen liegenden Bereichen der Fenster vorgesehen. Diese Bauweise ist für alle vorgehend bzw. nachfolgend beschriebenen Ausführungsformen optional möglich.

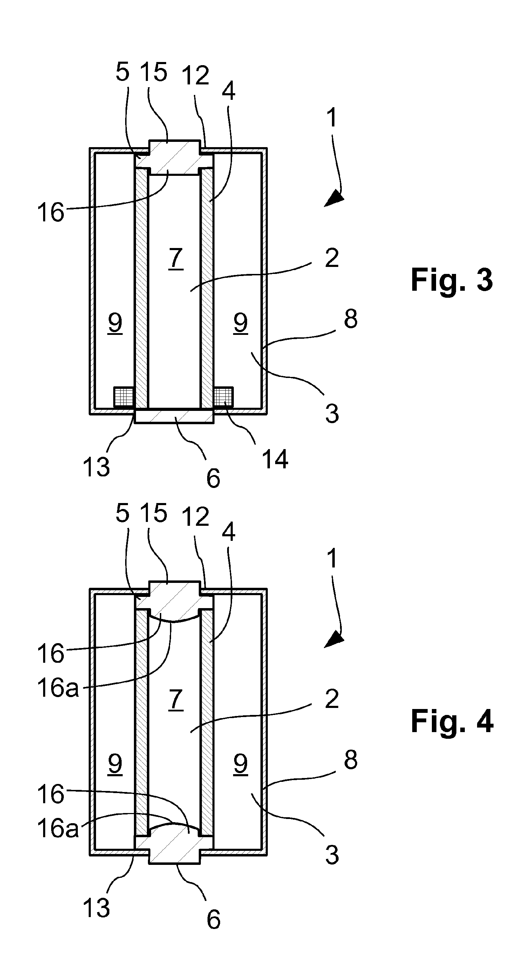

Fig. 3 zeigt zwei weitere mögliche Befestigungsmöglichkeiten bzw. Ausbildungen der Sterilisierungshülle 3 im Bereich der Fenster 5 und 6. Am Fenster 6 ist zur besseren Befestigung des Hüllenrandes 13 ein Montagering 14 vorgesehen, der an der Wandung der Kapillare 4 außen angebracht ist. Natürlich kann der Montagering 14 auch am Rand des Fensters 5 liegen. Er stellt eine Montagefläche für den Hüllenrand 13 bereit.

In Fig. 3 ist weiter gezeigt, daß das Fenster 5 mit einem vom Meßkammerinnenraum 7 wegweisenden Außenvorsprung 15 versehen ist. Dadurch ist eine umlaufende Randnut oder -aussparung am Fenster 5 gebildet, an der der Hüllenrand 12 befestigt ist.

Zur Befestigung der Hüllenwand 8 der Sterilisierungshülle 3 können die anhand der Fig. 1 bis 3 exemplarisch geschilderten Bauweise in beliebiger Kombination eingesetzt werden. Auch andere Befestigungen am Rand der Fenster sind möglich.

Fig. 3 zeigt ein zusätzliches optionales Merkmal für eines der Fenster, im dargestellten Beispiel des Fensters 5. Es verfügt zusätzlich noch über einen Innenvorsprung 16. Dieser erstreckt sich in den Meßkammerinnenraum 7 am Bereich des Randes der Kapillare 4, genauer in dem Abschnitt, in dem das Fenster 5 auf die Stirnseite der Kapillare 3 aufgebracht ist. Verwendet man für die Verbindung zwischen Fenster und Stirnseite der Kapillare eine Verklebung, ist nahe dieser Stirnseite die Diffusionseigenschaft der Kapillare 4 verändert. Damit diese Veränderung nicht die Diffusion in den Meßkammerinnenraum 7 beeinflußt, sorgt der Innenvorsprung 16 dafür, daß in dem Bereich, in die Diffusionseigenschaften verändert sind, die Meßkammer 2 keinen Meßkammerinnenraum 7 hat, also keinen Bereich, in dem sich das Meßmedium befinden kann. Der Innenvorsprung 16 kann natürlich ganz unabhängig von der Art der Befestigung der Hüllenwand 8 an den Fenstern 5, 6 verwendet werden.

Fig. 4 zeigt eine abgewandelte Bauweise der Meßkammer der Fig. 3. Hier sind nun beide Fenster 5, 6 mit Innenvorsprüngen 16 versehen. Diese sind in Weiterbildung der in Fig. 3 für das Fenster 5 gezeigten Bauweise nun auch mit ihrer zum Meßkammerinnenraum 7 weisenden Fläche 16a konvex ausgebildet. Diese konvexe Fläche 16a bewirkt, daß eventuelle im Meßkammerinnenraum 7 verbliebene Gasbläschen nach außen gedrängt werden und so den optischen Strahlengang möglichst wenig stören. Weiter sind die konvexen Flächen 16a so ausgestaltet, daß aufgrund des Brechzahlenunterschiedes zwischen Material der Fenster 5, 6 und dem Meßmedium eine optische Wirkung erreicht wird, die zur besseren Fokussierung der Strahlung und/oder zur Vermeidung von störenden Randstrahlen eingesetzt wird.

Die Sterilisierungshülle 3 erlaubt eine Sterilisierung der Meßkammervorrichtung durch ionisierende Strahlung, ohne daß die noch zu erläuternden Sender- und Empfängervorrichtungen ebenfalls der ionisierenden Strahlung ausgesetzt werden müßten. Vor dem Betrieb eines Sensors mit der Meßkammervorrichtung 1 wird die Sterilisierungshülle 3 entfernt. Dazu kann beispielsweise ein geeigneter Aufreißfaden oder eine Sollbruchstelle in der Hüllenwand 8 vorgesehen werden. Auch können die Verbindungen des Hüllenrandes 12 oder 13 so ausgestaltet werden, daß sie oberhalb einer bestimmten Zugkraft aufgehen.

Die Wandung der Meßkammer 2 ermöglicht einen diffusiven Stoffausgleich zwischen der umgebenden Matrix und dem Meßmedium, bidirektional und stoffselektiv. Die Selektion ist dabei so gewählt, daß lediglich der nachzuweisende Stoff durch die Wandung des Gehäuses 2 diffundieren kann, oder zumindest einen um eine Größenordnung höheren Diffusionskoeffizienten hat, als Substanzen der Matrix.

Die Wandung läßt Substanzen mit einer Größe kleiner einer Maximalgröße in das Innere der Meßkammer 2, wohingegen Substanzen die größer sind, nicht durchgelassen werden. Ein beispielhafter Wert für die Maximalgröße ist z. B. 30 kDalton. Bei einem derart durch Behandlung bzw. Ausbildung der Wandlung erzeugten Größenselektionswert kann Glukose in die den Meßkammerinnenraum 7 diffundieren, größere Substanzen, die beispielsweise hinsichtlich des noch zu erläuternden Meßeffektes einen größeren Effekt zeigen würden, können hingegen nicht eindringen.

Die Meßkammervorrichtung 1 bewirkt eine dynamische und definierte Stoffselektion.

Fig. 5 zeigt schematisch die Meßkammervorrichtung 1 ergänzt um die Sendervorrichtung 17 und die Empfängervorrichtung 24. Die Sendervorrichtung 17 weist ein Fenster 18 auf, durch das optische Strahlung von einer Lichtquelle, im vorliegenden Beispiel eine Diode 19, läuft und an einem Fenster 18 ausgekoppelt wird. Die Diode 19 befindet sich auf einer Platine 20, die auch eine Optik 21 trägt, welche die Strahlung von der Diode 19 geeignet bündelt. Ein Gehäuse 22 trägt die Platine 20 und das Fenster 18. Auf das Gehäuse 22 ist optional eine Spitze 23 aufgesetzt, die zum besseren Einstechen des Sensors unter die Haut eines Säugers, beispielsweise eines Patienten, dessen Glukosespiegel im Blut gemessen werden soll, dient. Die beschriebene Ausgestaltung der Sendereinheit 17 ist exemplarisch. Wesentlich ist allerdings, daß sie zum Anschluß am Fenster 5 ausgebildet ist. Der Anschluß am Fenster 5 erfolgt in der in Fig. 5 gezeigten Bauweise dadurch, daß die Fenster 18 und 5 geeignet optisch miteinander gekoppelt werden. Verwendet man die bereits zuvor erwähnte Ausführung der Sendervorrichtung 17, die über eine Lichtleitfaser mit der Meßkammervorrichtung 1 verbunden wird, ist das Fenster 18 durch einen Faserkoppler ersetzt, und eine Lichtleitfaser führt von dort zum ebenfalls als Faserkoppler ausgeführten Fenster 5.

Die Bauweise der Fig. 5 zeigt dabei der einfacheren Darstellung halber eine Meßkammervorrichtung 1 gemäß Fig. 1. Natürlich sind die anderen Ausgestaltungen der Meßkammervorrichtung 1 gleichermaßen verwendbar, wobei die Sendervorrichtung 17 dann für die jeweilige Ausbildung des Fensters 5 (bzw. Faserkopplers) geeignet gestaltet ist.

Am gegenüberliegenden Fenster 6 ist die Empfängervorrichtung 24 angeordnet. Sie weist einen Strahlteilerwürfel 25 auf, der die durch die Meßkammer 2 gelaufene optische Strahlung zu zwei Detektoren 26, 27 aufteilt. Die Aufteilung erfolgt dabei entsprechend dem gewählten Meßprinzip, beispielsweise nach Polarisation, Wellenlänge etc. Für die Verbindung der Empfängervorrichtung 24 mit dem Fenster 6 gilt das für die Sendervorrichtung 17 und deren Anschluß an das Fenster 5 Gesagte gleichermaßen. Es ist möglich, den Strahlteilerwürfel bzw. ein damit optisch verbundenes Element direkt am Fenster 6 anzubringen. Alternativ kann eine Lichtleitfaserverbindung verwendet werden, indem das Fenster 6 als Faserkoppler ausgeführt wird, der über eine Lichtleitfaser an einen entsprechenden Faserkoppleranschluß an der Empfängervorrichtung 24 angebunden ist.

Soweit vorstehend oder nachfolgend vom "Anbringen" oder "Befestigen" der Sender- bzw. Empfängervorrichtung 17, 24 an die Meßkammervorrichtung 1 die Rede ist, ist deshalb damit im Falle einer Lichtleitfaseranbindung das optische Anschließen der Sender- bzw. Empfängervorrichtung 17, 24 an die Meßkammervorrichtung 1 gemeint.

Der von der Diode 19 ausgesandte optische Strahl 28 wird geeignet konditioniert (z. B. durch die Optik 21) und nach Durchqueren der Meßkammer 2 und etwaiger Wechselwirkung mit darin enthaltenen Stoffen direkt von der Empfängervorrichtung 24 erfaßt. In der Ausführungsform der Fig. 5 hängt die Schwächung des optischen Strahls 6 von der polarisationsabhängigen Absorption im Meßmedium ab. Die Absorption ist wiederum auf bekannte Art und Weise mit der Stoffkonzentration verknüpft, so daß aus der Intensität des optischen Strahls an den Detektoren 26, 27 und damit aus der Stärke der Empfängersignale automatisch ein Rückschluß auf die Stoffkonzentration erreicht ist. Die Bauweise der Fig. 5 eignet sich also besonders für Stoffe, welche die Absorption beeinflussen.

Sendervorrichtung 17 und Empfängervorrichtung 24 sind beide mit einer (nicht dargestellten) Steuerelektronik verbunden, welche einerseits die Diode 19 ansteuert und andererseits die Detektoren 26, 27 ausliest.

Fig. 5 zeigt den Sensor in einem lagerfähigen Zustand. Vor dem Einstechen wird natürlich die Sterilisierungshülle bzw. deren Hüllenwand 8 entfernt.

Im Herstellprozeß ist zu dem Zeitpunkt, zu dem die Sendervorrichtung 17 und die Empfängervorrichtung 24 an der Meßkammervorrichtung 1 angebracht werden, diese bereits im Inneren steril, weil mittels ionisierender Strahlung sterilisiert. Der Sensor, wie in Fig. 5 zu sehen ist, kann nach dem Anbringen der Sendervorrichtung 17 und der Empfängervorrichtung 24 einfach oberflächensterilisiert werden, beispielsweise durch eine sogenannte chemische Sterilisierung mittels Gas etc.

Die Optik 21, welche für die Sendervorrichtung 17 eingezeichnet ist, ist lediglich exemplarisch als Kollimatoroptik eingezeichnet. Sie kann beispielsweise ein Filtersystem, wie Polarisationsfilter, Interferenzfilter oder Kantefilter zusätzlich oder alternativ umfassen. Die Ausgestaltung des optischen Filtersystems hängt von der Art des anzuwendenden Meßverfahrens ab. Natürlich ist vorzugsweise auch ein zum senderseitigen optischen Filtersystem passendes optisches Empfängerfiltersystem in der Empfängereinrichtung 24 angeordnet. Damit ist ein passendes Meßverfahren möglich, das über die polarimetrische Differenzmessung, welche mit dem geschilderten Aufbau der Fig. 5 realisierten natürlich hinausgehen kann.

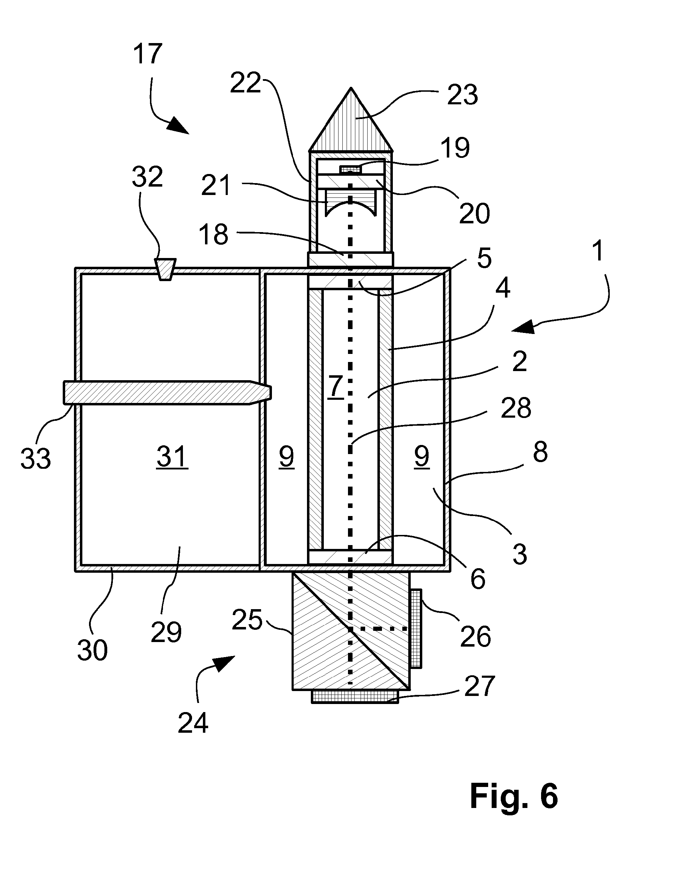

Fig. 6 zeigt einen Sensor mit weitergebildeter Meßkammervorrichtung 1. Sie umfaßt zusätzlich noch eine Kalibrierhülle 29, die im Beispiel durch eine Hüllenwand 30 gebildet ist, welche an die Hüllenwand 8 der Sterilisierungshülle 3 angefügt ist. Die Kalibrierungshülle 29 stellt einen Hülleninnenraum 31 bereit, der mit einem Kalibriermittel gefüllt ist. Der Hüllenraum 31 ist zum einen über einen Fluidanschluß 32 befüllt. Es kann sich dabei um die gleiche Art der Befüllung handeln, wie sie anhand der Meßkammer 2 und der Sterilisierungshülle 3 beschrieben wurde.

Zum anderen ist eine Fluidverbindung 33 zwischen dem Hülleninnenraum der Kalibrierhülle 29 und Hülleninnenraum 9 der Stabilisierungshülle vorgesehen. Diese Fluidverbindung kann selektiv geöffnet werden, so daß sich das Kalibriermedium im Hülleninnenraum 31 mit dem Meßmedium im Hüllenraum 9 vermischt. Dies ermöglicht es, wie im allgemeinen Beschreibungsteil bereits erläutert, einen zweiten Meßpunkt zum Kalibrieren des Sensors zu erzeugen. Zur Kalibrierung wird zuerst der Sensor mit nicht hergestellter Fluidverbindung betrieben. Das Meßmedium im Meßkammerinnenraum 7 und die darin enthaltene Konzentration des zu messenden Stoffes liefert den ersten Kalibrierpunkt. Anschließend wird die Fluidverbindung 33 hergestellt, z. B. geöffnet, so daß sich das Kalibriermedium aus dem Hülleninnenraum 31 mit dem Meßmedium aus dem Hülleninnenraum 9 vermischt. Aufgrund der Diffusionseigenschaften der Kapillare 4 findet damit eine durch das Kalibriermedium bewirkte Veränderung der Stoffzusammensetzung im Meßkammerinnenraum 7 statt. Dies liefert einen zweiten Kalibrierpunkt.

Die Kalibrierhülle 29 ist in der Darstellung der Fig. 6 exemplarisch so ausgebildet, daß sie an die Sterilisierungshülle 3 angesetzt ist, sich also die Sterilisierungshülle 3 und die Kalibrierhülle 29 eine Wandung teilen. Dies ist jedoch nicht zwingend erforderlich. Die Kalibrierhülle 29 kann auch als eigenständige Hülle, welche das Kalibriermedium einschließt, vorgesehen werden. Entscheidend ist lediglich, daß eine Fluidverbindung zwischen der Kalibrierhülle 29 und der Sterilisierungshülle 3 herstellbar ist, über die dafür gesorgt werden kann, daß das Kalibriermedium mit dem Meßmedium vermischt werden kann.

Diese Fluidverbindung kann auch durch eine Einstichöffnung an der Sterilisierungshülle 3 realisiert werden, so daß die Kalibrierhülle dann durch eine das Kalibriermedium enthaltende Spritze mit entsprechender Kanüle realisiert ist.

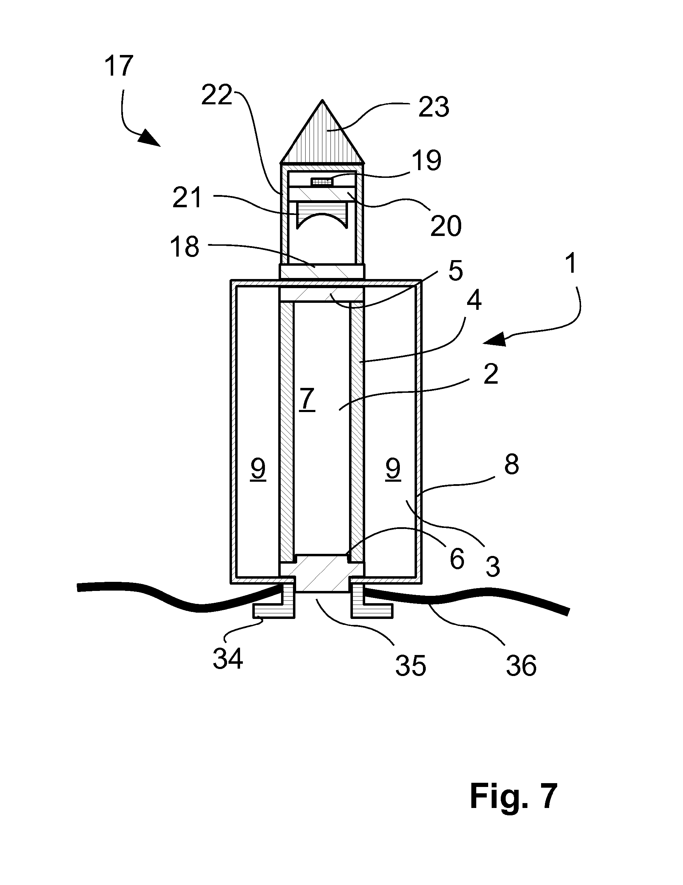

Fig. 7 zeigt in einer schematischen Darstellung ein Modul für einen Sensor zum Erfassen der Stoffkonzentration, der nach der beschriebenen Art arbeitet. Das Modul besteht aus der Meßkammervorrichtung 1 und der Sendervorrichtung 17, die beide gemäß den vorliegend geschilderten Prinzipien aufgebaut sind. Zum Anbringen der Empfängervorrichtung 24 ist am entsprechenden Fenster 6 der Meßkammervorrichtung 1 ein Flansch 34 angebracht, an dem die Empfängervorrichtung 24 befestigt werden kann. Um den Durchtritt der optischen Strahlung vom Fenster 6 zur Empfängervorrichtung 24 möglichst vollständig zu erreichen, weist der Flansch 34 ein Volumen 35 auf, in das eine Immersionsflüssigkeit, beispielsweise ein Immersionsöl, eingebracht werden kann, bevor die Empfängervorrichtung 24 am Flansch 34 befestigt wird.

Die Ausbildung bzw. Verwendung eines Flansches ist natürlich nur eine von mehreren Möglichkeiten, Mittel zur Befestigung der Empfängereinheit 24 vorzusehen. Ein wesentlicher Aspekt der Bauweise der Fig. 7 liegt darin, daß ein Modul bereitgestellt wird, das diejenigen Elemente des Sensors umfaßt, die später zumindest teilweise in den Körper, in den die Gewebeflüssigkeit hinsichtlich einer Stoffkonzentration erfaßt werden soll, eingebracht wird. Die Mittel zur Befestigung, beispielsweise in Form des Flansches 34 erlauben eine einfache Befestigung derjenigen Elemente, d. h. der Empfängereinheit 24, die nicht in den Körper inkorporiert werden. Durch das Modul wird ein kostengünstiger Dauerbetrieb des Sensors erreicht, da nur das Modul ausgetauscht werden muß, die Empfängereinheit 24 jedoch nicht.

Zum Befestigen des Sensors nach Inkorporation des Moduls in den Körper ist am Fenster 6 eine Befestigungs- und Schutzvorrichtung vorgesehen, die beispielsweise in Form einer Manschette 36 ausgebildet sein kann. Die Befestigungs- und Schutzvorrichtung ist einerseits am Modul fest angebracht und andererseits flexibel ausgebildet. Sie kann mit der Haut des Körpers, in dem das Modul eingebracht wird, verbunden werden, beispielsweise durch eine chirurgische Naht, eine Verklebung, Pflaster etc. Dadurch ist zum einen sichergestellt, daß das inkorporierte Modul Körperbewegungen einfach folgen kann, d. h. also die Belastung für einen Patienten gering ist. Zum anderen ist das inkorporierte Modul und insbesondere die Durchtrittsstelle durch die Haut vor Infektionen etc. geschützt. Die Schutz- und Befestigungsvorrichtung, beispielsweise die Manschette 36, ist dabei bevorzugt für optische Strahlung intransparent, so daß keine störende Strahlung seitlich der Meßkammervorrichtung 1 zur Empfängervorrichtung 24 gelangt. Auch ist natürlich verhindert, daß durch die Haut störende Strahlung zur Meßkammervorrichtung 1 gelangen kann, die zu einem Störsignal an der Empfängervorrichtung 24 führen könnte.

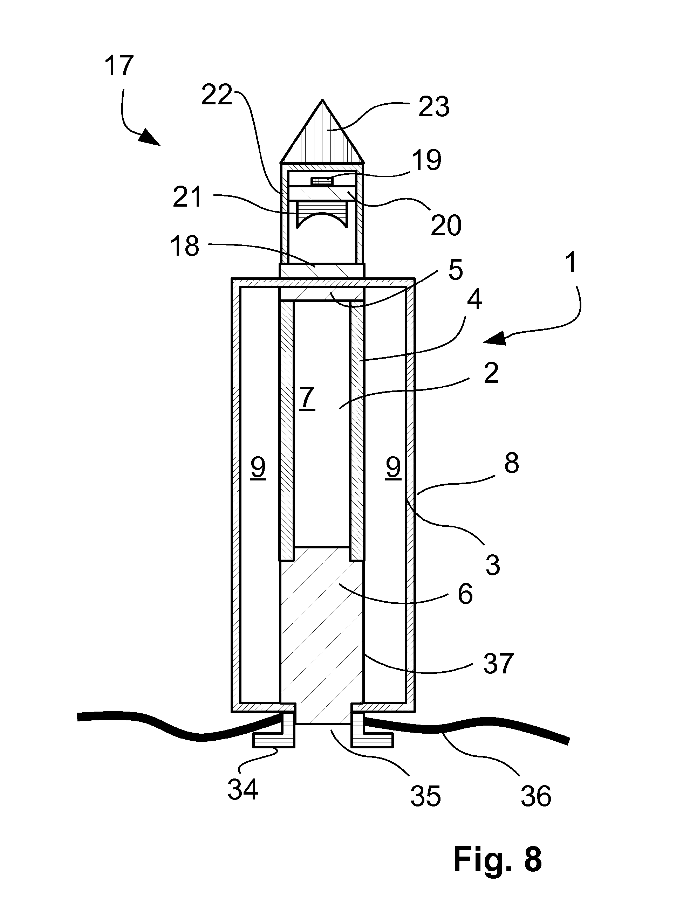

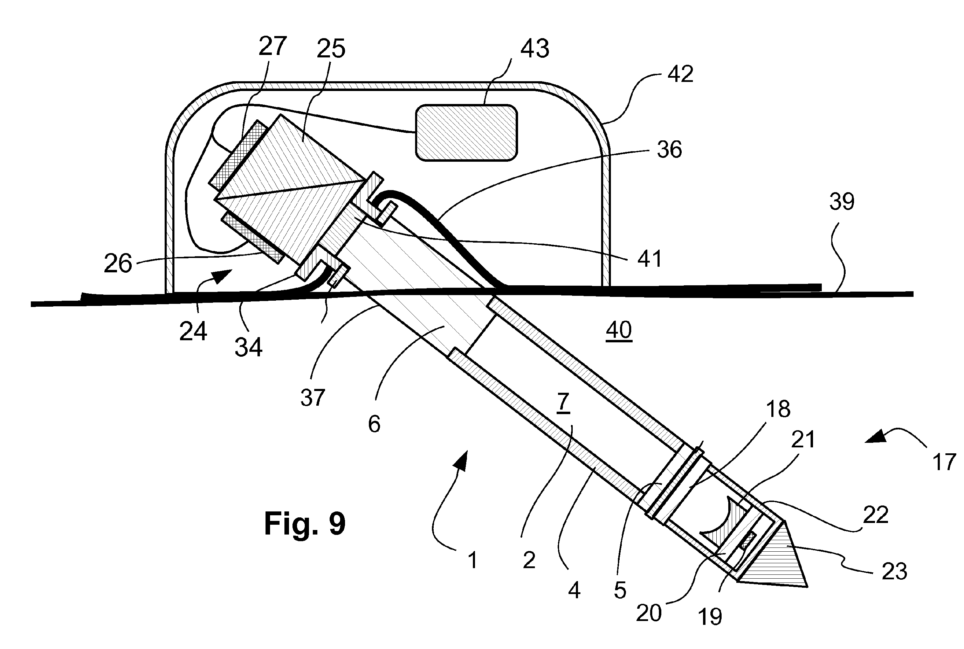

Fig. 8 zeigt eine Abwandlung der Meßkammervorrichtung 1, hier exemplarisch für das Modul der Fig. 7. Das Fenster 6, an dem die Empfängervorrichtung 24 angebracht wird, ist hier mit einer Verlängerung 37 ausgestattet. Hierdurch kommt auch bei einem schrägen Einstich des Moduls bzw. des Sensors unter die Haut die gesamte Kapillare 4 in einen Bereich des Körpers, in dem Gewebeflüssigkeit vorliegt. Dies zeigt sich exemplarisch an der Schemadarstellung der Fig. 9, die den Sensor schräg unter eine Haut 39 eingestochen darstellt. Natürlich wurde vor dem Einstechen die Hüllenwand 8 der Sterilisierungshülle 3 entfernt. Je nach Befestigung der Hüllenwand 8 im Bereich der Fenster 5 und 6 kann dabei ein Hüllenrest 38 stehenbleiben, der bei der in Fig. 9 exemplarisch gezeigten Bauweise mit einem Hüllenfenster am Fenster 5 sogar im optischen Strahlengang verbleibt, dort aber nicht stört.

Fig. 9 zeigt weiter die am Flansch 34 befestigte Empfängervorrichtung 24. Wie zu sehen ist, ist zwischen der Empfängervorrichtung 24 und dem Fenster 6 eine Immersionsflüssigkeit 41 eingebracht, die einen möglichst vollständigen Durchtritt der Strahlung vom Fenster 6 zur Empfängervorrichtung 24 gewährleistet. Die Empfängervorrichtung 24, beispielsweise die Detektoren 26 und 27 sind mit einer Elektronik 43 verbunden, welche die entsprechende Signalauslesung bewirkt.

Der Sensor ist einerseits mittels seiner Manschette 36 an der Haut 39 befestigt. Die Flexibilität der Manschette 36 bewirkt, daß der eingestochene Sensor Körperbewegungen folgen kann. Gleichzeitig ist der aus dem Körper ragende Teil des Sensors mit einer Schutzkappe 42 abgedeckt, die unabhängig von der Manschette 36 an der Haut 39 befestigt ist. Die Elektronik 43 ist an der Schutzkappe 42 befestigt, und die Verbindungen zwischen Elektronik 43 und Empfängervorrichtung 24 bzw. Sensorvorrichtung 17 sind flexibel. Dadurch ist sichergestellt, daß ein Druck etc. auf die Schutzkappe 42 nicht zu einer Bewegung derjenigen Teile des Sensors führt, die in den Körper inkorporiert sind. Gleichzeitig schützt die Schutzkappe 42 die au dem Körper ragenden Teile des Sensors und trägt die Elektronik 43.

Zum Auswechseln der in den Körper ragenden Teile des Sensors, was nach etwa 14 Tagen üblicherweise erforderlich ist, wird die Schutzkappe 42 abgenommen, wozu geeignete elektrische Steckverbindungen zum Sensor gelöst werden. Dann wird die Empfängervorrichtung 24 an ihrer Verbindung zum Fenster 6 gelöst. Dann kann das Modul bestehend aus Meßkammervorrichtung 1 und Empfängervorrichtung aus dem Körper herausgezogen werden und durch ein frisches Modul ersetzt werden, an dem dann wieder die Empfängereinheit 23 befestigt wird. Somit ist eine kostengünstige Auffrischung des Sensors möglich, ohne die Empfängereinheit 24 neu bereitstellen zu müssen.

## Patentansprüche

1. Meßkammervorrichtung für einen optisch arbeitenden Sensor zum Bestimmen einer Konzentration eines Stoffes, der in Gewebeflüssigkeit (40) eines Säugers enthalten ist, wobei die Meßkammervorrichtung (1) eine Meßkammer (2) aufweist, die zum Einsetzen in den Körper des Säugers ausgebildet ist, mit einem flüssigen Meßmedium befüllt ist und eine Wandung (4) aufweist, welche zumindest in einem Wandungsabschnitt für den Stoff besser diffusionsdurchlässig ist, als für andere Bestandteile der Gewebeflüssigkeit (40),
**dadurch gekennzeichnet, daß**
- die Meßkammer (2) aufweist
- einen ersten Meßkammerfensterabschnitt (5), der zum Verbinden mit einer Sendervorrichtung (17) zur Abgabe optischer Strahlung in die Meßkammer (2) vorbereitet ist, und
- einen zweiten Meßkammerfensterabschnitt (6), der zum Verbinden mit einer Empfängervorrichtung (24) zum Empfang von durch die Meßkammer (2) gelaufener optischer Strahlung vorbereitet ist, wobei
- die Wandung (4) und die Meßkammerfensterabschnitte (5, 6) das Meßmedium tropfdicht einschließen,
- die Meßkammervorrichtung (1) eine Sterilisierungshülle (3) aufweist, wobei
- die Sterilisierungshülle (3) zumindest den Wandungsabschnitt (4) der Meßkammer (2) einhüllt, jedoch ohne die Meßkammerfensterabschnitte (5, 6) für die optische Strahlung zu verschließen und für das Verbinden mit Sendervorrichtung (17) bzw. Empfängervorrichtung (24) zu blockieren,
- die Sterilisierungshülle (3) ebenfalls mit dem Meßmedium gefüllt ist, um den Wandungsabschnitt (4) mit dem Meßmedium zu umgeben, und
- die Sterilisierungshülle (3) vor dem Einsatz der Meßkammer (2) entfernbar ist.

2. Meßkammervorrichtung nach Anspruch 1, **dadurch gekennzeichnet, daß** die Sterilisierungshülle (3) an die Meßkammerfensterabschnitte (5, 6) umgebenden Bereichen der Meßkammer (2) dichtend angebracht ist und die Meßkammerfensterabschnitte (5, 6) nicht überdeckt, oder, daß die Sterilisierungshülle (3) die Meßkammerfensterabschnitte (5, 6) überdeckt und für die optische Strahlung transparent ist.

3. Meßkammervorrichtung nach einem der obigen Ansprüche, **dadurch gekennzeichnet, daß** die Sterilisierungshülle (3) eine Folie (8) aufweist, die insbesondere einen Aufreißfaden zum Entfernen der Sterilisierungshülle (3) hat.

4. Meßkammervorrichtung nach einem der obigen Ansprüche, **dadurch gekennzeichnet, daß** die Meßkammerfensterabschnitte (5, 6) mit der Wandung (4) verklebt sind und in die Meßkammer (2) hineinragen und Bereiche der Wandung (4) überdecken, in denen eine Diffusionspermeabilität der Wandung (4) durch die Verklebung herabgesetzt ist.

5. Meßkammervorrichtung nach einem der obigen Ansprüche, **dadurch gekennzeichnet, daß** am ersten Meßkammerfensterabschnitt (5) und/oder zweiten Meßkammerfensterabschnitt (6) Befestigungsmittel (34) zum Befestigen der anzuschließenden Sendervorrichtung (17) bzw. Empfängervorrichtung (24) vorgesehen sind, insbesondere einen Flansch umfassende Befestigungsmittel, wobei optional die Befestigungsmittel (34) ein Reservoir (35) für eine Immersionsflüssigkeit (41) aufweisen.

6. Meßkammervorrichtung nach einem der obigen Ansprüche, **dadurch gekennzeichnet, daß** die Sterilisierungshülle (3) mit einer Kalibrierkammer (29) über eine Fluidverbindung (33) verbunden ist, wobei die Kalibrierkammer (29) ein Kalibriermittel enthält und die Fluidverbindung (33) verschlossen aber öffenbar ist, um für Kalibrierzwecke das Kalibriermittel mit dem Meßmittel zu mischen.

7. Meßkammervorrichtung nach einem der obigen Ansprüche, **dadurch gekennzeichnet, daß** am Rand des ersten und/oder zweiten Meßkammerfensterabschnittes (5, 6) ein Befestigungsmittel (36) angebracht ist, mit dem die Meßkammervorrichtung (1) nach Einsetzen in den Körper des Säugers an einer Haut des Säugers befestigbar ist, wobei das Befestigungsmittel insbesondere als elastische Manschette (36) ausgebildet ist.

8. Meßkammervorrichtung nach einem der obigen Ansprüche, **dadurch gekennzeichnet, daß** der erste und/oder zweite Meßkammerfensterabschnitt (5, 6) eine zum Meßkammerinneren (7) weisende Fensterfläche (16a) hat, die konvex ist, um im Meßmedium befindliche Gasbläschen an den Rand des/der Meßkammerfensterabschnitt(e) (5, 6) zu drängen.

9. Meßkammervorrichtung nach einem der obigen Ansprüche, **dadurch gekennzeichnet, daß** der erste und/oder zweite Meßkammerfensterabschnitt (5, 6) als Faserkoppler ausgebildet sind/ist oder Faserkoppler aufweisen/einen Faserkoppler aufweist, um die Sender- bzw. Empfängervorrichtung (17, 24) über eine Lichtleitfaser anzuschließen.

10. Modul für einen Stoff-Konzentrationssensor, wobei das Modul **gekennzeichnet ist durch** eine Meßkammervorrichtung (1) nach einem der obigen Ansprüche, wobei der erste Meßkammerfensterabschnitt (5) dem zweiten Meßkammerfensterabschnitt (6) gegenüberliegt, und die Meßkammer (2) zum Durchlauf der optischen Strahlung ohne Reflexion ausgebildet ist.

11. Stoff-Konzentrationssensor, **gekennzeichnet durch** eine Meßkammervorrichtung (1) nach einem der Ansprüche 1 bis 9, eine am ersten Meßkammerfensterabschnitt (5) angeschlossene Sendervorrichtung (17) und eine am zweiten Meßkammerfensterabschnitt (6) angeschlossene Empfängervorrichtung (24).

12. Sensor nach Anspruch 11, **gekennzeichnet durch** eine über die Empfängervorrichtung (24) anordenbare Schutzkappe (42), wobei Mittel zum elastischen Verbinden der Schutzkappe (42) mit der Empfängervorrichtung (24) vorgesehen sind und wobei optional die Schutzkappe (42) von der Meßkammervorrichtung (1) unabhängige Mittel zum Befestigen an einer Haut des Säugers aufweist.

13. Verfahren zum Herstellen einer Meßkammervorrichtung (1) nach einem der Ansprüche 1 bis 9, **dadurch gekennzeichnet, daß**
a) die Meßkammer (2) mit der Sterilisierungshülle (3) umgeben wird, wobei die Sterilisierungshülle (3) eine Öffnung aufweist,
b) die Einheit aus Meßkammer (2) und Sterilisierungshülle (3) unter Einsatz von Vakuum mit dem Meßmedium befüllt wird und die Öffnung verschlossen wird und
c) die befüllte Einheit mittels ionisierender Strahlung sterilisiert wird.

14. Verfahren zum Herstellen eines Moduls nach Anspruch 10, **dadurch gekennzeichnet, daß** eine Messkammervorrichtung nach einem der Ansprüche 1 bis 9 mittels des Verfahrens nach Anspruch 13 hergestellt wird und nach Schritt c) weiter eine Sendervorrichtung (17) mit dem ersten Meßkammerfensterabschnitt (5) verbunden und anschließend eine Oberflächensterilisierung durchgeführt wird.

15. Verfahren zum Herstellen eines Sensors nach Anspruch 11, **dadurch gekennzeichnet, daß** zuerst das Verfahren nach Anspruch 14 ausgeführt wird, wobei vor der Oberflächensterilisierung die Empfängervorrichtung (24) mit dem zweiten Meßkammerfensterabschnitt (6) verbunden wird und wobei eine Meßkammervorrichtung (1) nach Anspruch 6 verwendet wird und vor dem Einsetzen des Sensors in den Körper des Säugers der Sensor kalibriert wird, indem eine Messung vor Entfernen der Sterilisierungshülle (3) und vor Öffnen der Fluidverbindung (33) durchgeführt wird und anschließend die Fluidverbindung (33) geöffnet wird und eine Messung bei mit dem Kalibriermittel gemischtem Meßmittel durchgeführt wird.

## Claims

1. Measurement chamber device for an optical sensor for measuring the concentration of a substance which is part of an interstitial fluid (40) of a mammal, wherein the measurement chamber device (1) comprises a measurement chamber (2), which is designed to be inserted into the body of the mammal, which is filled with a measurement fluid and which contains a wall (4), which enables at least in a wall section a better diffusion of the substance than for other components of the interstitial fluid (40), **characterized in that**
- the measurement chamber (2) comprises
- a first measurement chamber window section (5), which is prepared to be connected to a transmitter unit (17) for emitting optical radiation into the measurement chamber (2), and
- a second measurement chamber window section (6), which is prepared to be connected to a receiver unit (24) for detecting optical radiation transmitted through the measurement chamber (2), wherein
- the wall (4) and the measurement window sections (5, 6) enclose the measurement fluid leakproof,
- the measurement chamber device (1) comprises a sterilization envelope (3), wherein
- the sterilization envelope (3) encloses the wall section (4) of the measurement chamber (2), but does not block the optical radiation at the measurement chamber window sections (5, 6) and does not obstruct to connect the transmitter unit (17) or the receiver unit (24),
- the measurement chamber envelope (3) is filled also with measurement fluid to enclose the wall section (4) in measurement fluid and
- the sterilization envelope (3) is removable before use of the measurement chamber (2).

2. The measurement chamber device according to claim 1, **characterized in that** the sterilization envelope (3) is mounted sealingly to portions of the measurement chamber (2) which are adjacent to the measurement chamber window sections (5, 6) and does not overlap with the measurement chamber window sections (5, 6) or that the sterilization envelope (3) overlaps with the measurement chamber window sections (5, 6) and is transparent for the optical radiation.

3. The measurement chamber device according to any of the above claims, **characterized in that** the sterilization envelope (3) comprises a sheet (8), which optional has an opening thread to remove the enclosing envelope (3).

4. The measurement chamber device according to any of the above claims, **characterized in that** the measurement chamber window sections (5, 6) are glued to the wall (4) and extend into the measurement chamber (3) and cover sections of the wall (4), at which a diffusion permeability of the wall (4) is reduced due to the gluing.

5. The measurement chamber device according to any of the above claims, **characterized in that** a mounting portion (34) is provided at the first measurement chamber window section (5) and/or the second measurement chamber section (6) to mount the transmitter unit (17) and/or the receiver unit (24), wherein the mounting portion may include a flange and wherein optional the mounting portion (34) provides a reservoir (35) for an immersion fluid (41).

6. The measurement chamber device according to any of the above claims, **characterized in that** the sterilization envelope (3) is connected to a calibration chamber (29) via a fluid connection (33), wherein the calibration chamber (29) contains a calibration fluid and the fluid connection (33) is closed and can be opened for calibration purposes to mix the measurement fluid with the calibration fluid.

7. The measurement chamber device according to any of the above claims, **characterized in that** a mounting part (36) is attached to the first and/or second measurement window section (5, 6) to enable attachment to the skin of the mammal once the measurement chamber device (1) has been inserted into the body of the mammal, which mounting part can be realized by an elastic sleeve (36).

8. The measurement chamber device according to any of the above claims, **characterized in that** the first and/or second measurement window sections (5, 6) comprise a window surface (16a) having a convex form at the inside of the measurement chamber (7) to push any gas bubbles in the measurement fluid to the periphery of the measurement chamber window section(s) (5, 6).

9. The measurement chamber device according to any of the above claims, **characterized in that** the first and/or second measurement window section (5, 6) is realized as a fiber coupler or comprises a fiber coupler to connect the transmitter and/or receiver unit (17, 24) by an optical fiber.

10. A module for a sensor for measuring a concentration of a substance, which module is **characterized by** a measurement chamber device (1) according to any of the above claims, wherein the first measurement chamber window section (5) is located opposite the second measurement chamber window section (6) and the measurement chamber (2) is adapted for transmission of the optical radiation without reflection.

11. A sensor for measuring a concentration of a substance, which sensor is **characterized by** a measurement chamber device (1) according to any of claims 1 to 9 by a receiver unit (17) connected to the first measurement chamber window section (5) and by a receiver unit (24) connected to the second measurement chamber window section (6).

12. The sensor of claim 11, which is **characterized by** a protection cap (42) to be placed over the receiver unit (24), which protection cap (42) comprises an elastic connection to the receiver unit (24), wherein optionally the protection cap (42) comprises an attaching part to attach the cap to the skin of the mammal, which attaching part is independent from the measurement chamber device (1).

13. A method for producing a measurement chamber device (1) according to any of claims 1 to 9, **characterized in that**
a) the measurement chamber (2) in enclosed by the sterilization envelope (3), wherein the sterilization envelope (2) comprises a port, and
b) the unit of measurement chamber (2) and sterilization envelope (3) is filled with the measurement fluid under vacuum conditions and the port is closed, and
c) the filled unit is sterilized by utilizing ionizing radiation.

14. A method for producing of a module of claim 10, **characterized in that** a measurement chamber device (1) according to any of claims 1 to 9 is produced according to the method of claim 13 and that after step c) the transmitter unit (17) is connected to the measurement window section (5) and then sterilized by a surface sterilization.

15. A method for producing a sensor according to claim 11, **characterized in that** first the method of claim 14 is performed, wherein prior to surface sterilization the receiver unit (24) is connected to the second measurement chamber window section (6) and wherein a measurement chamber device (1) according to claim 6 is used and wherein prior to insertion of the sensor into the body of the mammal the sensor is calibrated by a first measurement which is preformed before removal of the sterilization envelope (3) and before opening of the fluid connection (33) and by a second measurement which is performed after opening of the fluid connection (33) resulting in the measurement fluid being mixed with the calibration fluid.

## Revendications

1. Dispositif de chambre de mesure pour un capteur optique pour la détermination de la concentration d'une substance contenue dans le liquide tissulaire (40) d'un aspirateur, le dispositif de chambre de mesure (1) comprenant une chambre de mesure (2) qui est conçue pour être insérée dans le corps de l'aspirateur, remplie avec un fluide de mesure liquide et comprend une paroi (4) qui est mieux perméable à la diffusion pour la substance, dans une portion de la paroi, que d'autres composants du liquide tissulaire (40),
**caractérisé en ce que**
- la chambre de mesure (2) comprend
- une première portion de fenêtre de chambre de mesure (5), préparée à la liaison avec un dispositif de capteur (17) pour l'émission d'un rayonnement optique vers la chambre de mesure (2) et
- une deuxième portion de fenêtre de chambre de mesure (6), préparée à la liaison avec un dispositif de réception (24) pour la réception d'un rayonnement optique traversant la chambre de mesure (2),
- la paroi (4) et les portions de fenêtres de chambre de mesure (5, 6) englobent le fluide de mesure de manière étanche aux gouttes,
- le dispositif de chambre de mesure (1) comprend une enveloppe de stérilisation (3),
- l'enveloppe de stérilisation (3) enveloppant au moins la portion de paroi (4) de la chambre de mesure (2), mais sans fermer les portions de fenêtres de chambre de mesure (5, 6) au rayonnement optique ni les bloquer pour la liaison avec le dispositif de capteur (17) ou le dispositif de réception (24),
- l'enveloppe de stérilisation (3) est également remplie du fluide de mesure afin d'entourer la portion de paroi (4) avec le fluide de mesure et
- l'enveloppe de stérilisation (3) peut être retirée avant l'insertion de la chambre de mesure (2).

2. Dispositif de chambre de mesure selon la revendication 1, **caractérisé en ce que** l'enveloppe de stérilisation (3) est montée de manière étanche sur les zones de la chambre de mesure (2) entourant les portions de fenêtres de chambre de mesure (5, 6) et ne recouvre pas les portions de fenêtres de chambre de mesure (5, 6) ou **en ce que** l'enveloppe de stérilisation (3) recouvre les portions de fenêtres de chambre de mesure (5, 6) et est transparente pour le rayonnement optique.

3. Dispositif de chambre de mesure selon l'une des revendications précédentes, **caractérisé en ce que** l'enveloppe de stérilisation (3) comprend un film (8) qui comprend plus particulièrement un fil de déchirement pour le retrait de l'enveloppe de stérilisation (3).

4. Dispositif de chambre de mesure selon l'une des revendications précédentes, **caractérisé en ce que** les portions de fenêtres de chambre de mesure (5, 6) sont collées avec la paroi (4) et dépassent dans la chambre de mesure (2) et recouvrent des zones de la paroi (4) dans lesquelles une perméabilité de diffusion de la paroi (4) est diminuée par le collage.

5. Dispositif de chambre de mesure selon l'une des revendications précédentes, **caractérisé en ce que**, au niveau de la première portion de fenêtre de chambre de mesure (5) et/ou de la deuxième portion de fenêtre de chambre de mesure (6), des moyens de fixation (34) sont prévus pour la fixation du dispositif de capteur (17) ou du dispositif de réception (24) à raccorder, plus particulièrement un moyen de fixation comprenant une bride, les moyens de fixation (34) comprenant en option un réservoir (35) pour un fluide d'immersion (41).

6. Dispositif de chambre de mesure selon l'une des revendications précédentes, **caractérisé en ce que** l'enveloppe de stérilisation (3) est reliée à une chambre de calibrage (29) par l'intermédiaire d'une liaison fluidique (33), la chambre de calibrage (29) contenant un fluide de calibrage et la liaison fluidique (33) étant fermée mais pouvant être ouverte afin de mélanger le fluide de calibrage avec le fluide de mesure pour des fins de calibrage.

7. Dispositif de chambre de mesure selon l'une des revendications précédentes, **caractérisé en ce que**, au bord de la première et/ou de la deuxième portion de fenêtre de chambre de mesure (5, 6), est monté un moyen de fixation (36) avec lequel le dispositif de chambre de mesure (1) peut être fixé, après l'insertion dans le corps de l'aspirateur, à une coque de l'aspirateur, le moyen de fixation étant conçu plus particulièrement comme une manchette élastique (36).

8. Dispositif de chambre de mesure selon l'une des revendications précédentes, **caractérisé en ce que** première et/ou la deuxième portion de fenêtre de chambre de mesure (5, 6) comprend une surface de fenêtre (16a) orientée vers l'intérieur de la chambre de mesure (7), qui est convexe afin de pousser les bulles de gaz se trouvant dans le fluide de mesure vers le bord de la (des) portion(s) de fenêtre(s) de chambre de mesure (5, 6).

9. Dispositif de chambre de mesure selon l'une des revendications précédentes, **caractérisé en ce que** première et/ou la deuxième portion de fenêtre de chambre de mesure (5, 6) est (sont) conçue(s) comme un coupleur à fibres ou comprend/comprennent un (des) coupleur(s) à fibres pour raccorder le dispositif d'émission ou de réception (17, 24) par l'intermédiaire d'une fibre optique.

10. Module pour un capteur de concentration de substances, le module étant **caractérisé par** un dispositif de chambre de mesure (1) selon l'une des revendications précédentes, la première portion de fenêtre de chambre de mesure (5) étant située en face de la deuxième portion de fenêtre de chambre de mesure (6) et la chambre de mesure (2) étant conçue pour le passage du rayonnement optique sans réflexion.

11. Capteur de concentration de substances, **caractérisé par** un dispositif de chambre de mesure (1) selon l'une des revendications 1 à 9, un dispositif de capteur (17) raccordé à une portion de fenêtre de chambre de mesure (5) et un dispositif de réception (24) raccordée à une deuxième portion de fenêtre de chambre de mesure (6).

12. Capteur selon la revendication 11, **caractérisé par** un capuchon de protection (42) pouvant être disposé sur le dispositif de réception (24), des moyens étant prévus pour la liaison élastique du capuchon de protection (42) avec le dispositif de réception (24) et le capuchon de protection (42) comprenant, en option, des moyens indépendants du dispositif de chambre de mesure (1) pour la fixation à une coque de l'aspirateur.

13. Procédé de fabrication d'un dispositif de chambre de mesure (1) selon l'une des revendications 1 à 9, **caractérisé en ce que**
a) la chambre de mesure (2) est entouré par l'enveloppe de stérilisation (3), l'enveloppe de stérilisation (3) comprenant une ouverture,
b) l'unité constituée de la chambre de mesure (2) et de l'enveloppe de stérilisation (3) est remplie de fluide de mesure à l'aide de vide et l'ouverture est fermée et
c) l'unité remplie est stérilisée par un rayonnement ionisant.

14. Procédé de fabrication d'un module selon la revendication 10, **caractérisé en ce qu'**un dispositif de chambre de mesure selon l'une des revendications 1 à 9 est fabriqué à l'aide du procédé selon la revendication 13 et, après l'étape c), un dispositif d'émission (17) est en outre relié à la première portion de fenêtre de chambre de mesure (5) puis une stérilisation de surface est effectuée.

15. Procédé de fabrication d'un capteur selon la revendication 11, **caractérisé en ce que** le procédé est d'abord effectué selon la revendication 14, le dispositif de réception (24) relié avec la deuxième portion de fenêtre de chambre de mesure (6) avant la stérilisation de surface et un dispositif de chambre de mesure (1) selon la revendication 6 étant utilisé et, avant l'insertion du capteur dans le corps de l'aspirateur, un calibrage étant effectué, en effectuant une mesure avant le retrait de l'enveloppe de stérilisation (3) et avant l'ouverture de la liaison fluidique (33) puis la liaison fluidique (33) est ouverte et une mesure est effectuée lorsque le fluide de calibrage est mélangé avec le fluide de mesure.
